# EUROPEAN PATENT SPECIFICATION

(11) **EP 4 416 124 B1**
(45) Date of publication and mention of the grant of the patent: **03.09.2025**
(21) Application number: 21802577.3
(22) Date of filing: 14.10.2021
(51) Int. Cl.: C07C 37/50, C07C 39/04, C07C 39/07, C07C 39/08, C07C 15/073, C07C 6/08, C07C 15/02, C07C 5/27

(54) **PROCESS FOR PRODUCING CRESOL AND XYLENE**
VERFAHREN ZUR HERSTELLUNG VON KRESOL UND XYLOL
PROCÉDÉ DE PRODUCTION DE CRÉSOL ET DE XYLÈNE

(43) Date of publication of application: 21.08.2024
(73) Proprietor: UOP LLC, Des Plaines, Illinois 60017-5017 (US); China Petroleum & Chemical Corporation, Beijing 100728 (CN)
(72) Inventor: PHIVILAY, Somphonh Peter, Brookshire, Texas 77423 (US); RANE, Neelesh, Des Plaines, Illinois 60017 (US); ZHOU, Zhenhua, Katy, Texas 77494 (US)
(74) Representative: Keltie LLP
(86) International application number: PCT/US2021/071872
(87) International publication number: WO 2023/063973

(56) References cited:
- WO-A1-2020/162876
- GB-A- 1 232 027
- US-A- 4 149 019
- US-A- 4 605 790

## Description

### BACKGROUND OF THE INVENTION

Low and medium temperature coal tars typically contain large amounts of valuable oxygenate compounds, such as phenol, cresols, xylenols and trimethylphenols, along with less desirable longer chain alkylphenols (i.e., alkylphenols having an alkyl chain with 2 or more carbon atoms). Sometimes the content can be close to about 40 wt% of the coal tar stream. These phenols may be extracted from coal tar using various methods, such as washing with aqueous sodium hydroxide solution followed by neutralization with sulfuric acid or carbon dioxide, solvent extraction, pressurized crystallization, etc.

The composition of the crude phenols obtained from coal tar is very complicated. For example, the phenols mixture extracted from the fraction with boiling range from 170 to 240°C of one heavy coal tar contained 60 types of phenols, most of which have concentrations lower than 1 wt% of the whole coal tar, as disclosed Wang, et al., "Extraction and GC/MS analysis of phenolic compounds in low temperature coal tar from Northern Shaanxi", J. of China Coal Society, 36 (4) (2011), 664-669. Some of these phenols also have very similar boiling points. This makes their separation and purification extremely difficult. In addition, only certain phenols, such as phenol, cresols, xylenols, naphthols and possibly methylnaphthols, have high volumes, have been widely used, and are therefore of economic interest. US 10,723,675 and WO2020162877 describe a process for making phenol and xylenes from a coal-derived phenol-containing stream. Transalkylation of alkylphenols with benzene/toluene is used to make phenol and alkylbenzenes. The cresol and xylenols in the stream are converted to phenol, and the alkylbenzenes are converted to xylenes. WO2020162876 discloses a process for making cresols and optionally xylenes.

Transalkylation of propylphenol and benzene/toluene to phenol and alkylbenzenes using different zeolites, including HZSM-5, HBeta, and HMOR, was described in "Conversion of alkylphenol to phenol via transalkylation using zeolite catalysts," Yoshikawa et al., Catalysis Today, 347, 110-114, (2018).

The transalkylation reaction of 2,5-xylenol and phenol to make cresols occurred using different zeolite catalysts including HZSM-5, HBeta, and HMOR, was described in "Transalkylation of higher methylphenols with phenol to produce cresols and xylenols" Kopano Moeketsi, Doctoral Dissertation, Univ. of Cape Town, 2007.Therefore, there is a need for a method of producing cresol, and optionally xylene, from lower value longer chain alkylphenols in low and medium temperature coal tars.

### BRIEF DESCRIPTION OF THE DRAWINGS

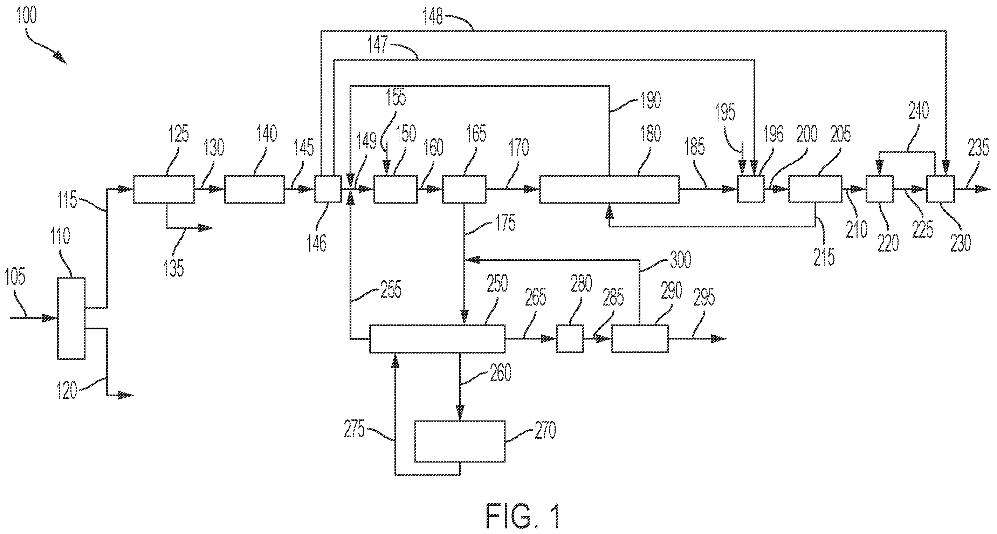

The Figure illustrates one embodiment of a process according to the present invention.

### DESCRIPTION OF THE INVENTION

The process of the disclosure provides a method of producing cresols and optionally xylenes.

The undesirable longer-chain alkylphenols in coal-derived feed streams can be converted to valuable products including, but not limited to, phenol, cresols, and xylenes by transalkylation. The alkylphenols in the coal-derived feed stream can be transalkylated with a reactant (e.g., benzene and/or toluene) to transfer the longer-chain alkyl group (for example, ethyl, propyl, butyl, etc.) to obtain phenol and alkylbenzenes (ethylbenzene, propylbenzene, ethyltoluene, etc.) as products. However, it has been determined that, in some cases, alkylphenols with methyl groups (such as cresols, xylenols, and trimethylphenols) can also be transalkylated with an aromatic compound, but not as easily as longer-chain alkyl groups.

Therefore, new processes have been developed which convert longer chain alkylphenols having two or more carbon atoms, as well as alkylphenols with methyl groups, into cresols. In some processes, xylenes may also be produced.

The process of the disclosure involves transalkylating the longer-chain alkylphenols (i.e., having an alkyl chain with 2 or more carbon atoms) with an aromatic solvent such as benzene and/or toluene in a first transalkylation reaction zone to obtain a reaction mixture comprising phenol, cresol, xylenol, trimethylphenol, and alkylbenzenes. Some xylenols and trimethylphenols can also convert under transalkylation to form cresols/phenol in the first transalkylation reaction zone. The remaining xylenols and trimethylphenols are reacted with phenol in a second transalkylation reactor to obtain the desired cresols. The aromatic solvent and alkylbenzene products from the first transalkylation reactor may be sent to an aromatic processing section to produce p-xylene. Unused aromatic solvent may be recycled to the first transalkylation reactor.

By using consecutive transalkylation reactors, the alkylphenols contained in the coal tar feed can be converted to more valuable cresols, and optionally xylenes.

The source of the alkylphenol stream can be a coal-derived feed stream, such as low temperature coal tar, medium temperature coal tar, high temperature coal tar, cresylic acid, or a crude phenolic mixture containing phenol, alkylphenols (methylphenols (cresols), ethylphenols, dimethylphenols (xylenols), propylphenols, butylphenols, methylethylphenols, trimethylphenols etc.), as well as heavier alkylphenols (such as indanols and naphthols). Coal tar is derived from the process of dry distillation and gasification of coal and is classified based on the temperature used for this process (400-600°C (low temperature), 600-1000°C (medium temperature), and greater than 1000°C (high temperature)). Cresylic acid is a generic term referring to combinations of phenol and alkylphenols, and it can be obtained from either coal or petroleum processing, for example. A crude phenolic mixture can be obtained by the processing of coal tar oils and the purification of phenol containing waste from coke ovens, low temperature carbonization, and hydrogenation plants, for example. The composition of the feed stream will vary depending on its source.

The coal-derived feed stream can be separated into various streams. Suitable separation processes include, but are not limited to, distillation, extraction, solvent extraction, acid and base extraction, adsorption, extractive distillation, crystallization, supercritical fluid extraction, chelation, eutectic reaction, or membrane separation. The crude alkylphenol stream may have a boiling point of about 300°C or less. For example, the crude alkylphenol stream may comprise a portion of a low or medium temperature coal tar distillate stream having a boiling point of about 300°C or less.

The separation effluent can optionally be treated to remove contaminants such as nitrogen, sulfur, and other elements. The treated fraction of the effluent can be used as the starting alkylphenol stream for the present process. Other methods of providing the alkylphenol stream could also be used. Optionally, phenol and cresols can be separated from the alkylphenol stream at this step.

The alkylphenol stream from the coal-derived feed stream comprises a complex mixture of alkylphenols comprising one or more methylphenols (cresols), ethylphenols, dimethylphenols (xylenols), trimethylphenols, propylphenols, butylphenols, methylethylphenols, etc.), as well as heavier alkylphenols (such as indanols and napthols).

The alkylphenol stream is transalkylated with benzene or toluene. Transalkylation is a chemical reaction resulting in transfer of an alkyl group from one organic compound to another. In the first transalkylation process, the alkylphenol stream and an aromatic reactant stream comprising one or more of benzene and toluene are provided to the first transalkylation reaction zone. The alkylphenol is transalkylated with benzene and/or toluene to form phenol and/or cresols and alkylbenzene and/or alkyltoluene. Catalysts, particularly zeolite catalysts, are often used to effect the reaction. If desired, the transalkylation catalyst may be metal stabilized using a noble metal or base metal, and may contain suitable binder or matrix material such as inorganic oxides and other suitable materials. The feed is usually heated to reaction temperature and then passed through a reaction zone, which may comprise one or more individual reactors or multiple catalyst beds in a single reactor. Passage of the combined feed through the reaction zone produces an effluent stream comprising unconverted feed and product monoalkylated hydrocarbons.

The transalkylation reaction may take place in any conventional or otherwise convenient manner and may comprise a batch or continuous type of operation, with continuous operation being preferred. The transalkylation catalyst is usefully disposed as a fixed bed in a reaction zone of a vertical tubular reactor, with the alkylaromatic feed stock charged through the bed in an upflow or downflow manner.

Any suitable transalkylation reaction conditions can be used. The transalkylation reaction conditions will depend on the particular reactor type use, as is known in the art. For the transalkylation of alkylphenol with benzene and/or toluene, the temperature is typically in the range of 50-600°C, or 100-500°C. The transalkylation zone is typically operated at pressures ranging from about 0 MPa(g) to 15 MPa(g), or 2 MPa(g) to 11 MPa(g). The WHSV is generally in the range of 0.1 to 300 hr⁻¹, or 1 to 250 hr⁻¹.

The catalyst is typically selected to have relatively high stability at a high activity level. Suitable transalkylation catalysts include, but are not limited to zeolites, acidic clay, silica alumina, acidic resins, mixed metal oxides, and the like, as are known in the art.

The molar ratio of benzene and toluene to alkylphenols may be in the range of 1-20.

The first transalkylation effluent stream may comprise one or more of cresol, phenol, xylenol, trimethylphenol, alkylbenzenes (e.g., xylene, ethylbenzene, and heavy alkylbenzenes (defined as alkylbenzenes containing 9 or more carbon atoms)), and alkylphenols having an alkyl chain of 2 or more carbon atoms.

The first transalkylation effluent stream is sent to the first transalkylation separation zone to be separated into a transalkylated alkylphenol stream comprising cresol, phenol, xylenol, trimethylphenol, and alkylphenols having an alkyl chain of 2 or more carbon atoms, and an aromatic stream comprising benzene, toluene, xylenes, ethylbenzene, and heavy alkylbenzenes.

The transalkylated alkylphenol stream is sent to a second transalkylation reaction zone where xylenols and trimethylphenols are transalkylated with phenol to produce cresol.

Any suitable second transalkylation reaction conditions can be used. The second transalkylation reaction conditions will depend on the particular reactor type use, as is known in the art. For the transalkylation of alkylphenol with phenol, the temperature is typically in the range of 50-600°C, or 100-500 °C. The transalkylation zone is typically operated at pressures ranging from about 0 MPa(g) to 15 MPa(g), or 0.01 MPa(g) to 10 MPa(g). The WHSV is generally in the range of 0.1 to 300 hr⁻¹, or 1 to 200 hr⁻¹.

The catalyst is typically selected to have relatively high stability at a high activity level. Suitable transalkylation catalysts include, but are not limited to zeolites, acidic clay, silica alumina, acidic resins, mixed metal oxides, and the like as are known in the art.

The molar ratio of phenol to xylenol/trimethylphenol may be in the range of 1 to 15.

The second transalkylation effluent stream comprises cresol, xylenol, trimethylphenol, and unconverted phenol. The second transalkylation effluent stream can be separated into a cresol stream comprising cresol, and a recycle phenol stream comprising xylenol, trimethyl phenol, and unconverted phenol. The cresol stream which is a mixture of cresols can be recovered.

The first and/or second transalkylation processes can be performed in any suitable reactor type, including but not limited to, fixed bed reactors, moving bed reactors, ebullated bed reactors, fluidized bed reactors, continuous catalyst regeneration (CCR) reactors, semi-regenerative reactors, batch reactors, continuous stirred tank (CSTR) reactors, and slurry bed reactors, or combinations thereof.

The cresol stream can be isomerized in a cresol isomerization reaction zone to form a mixed cresol stream. The mixed cresol stream can be separated to recover a specific cresol, such as m-cresol, and a second mixed cresol stream comprising o-cresol and p-cresol. The second mixed cresol stream can then be recycled back to the cresol isomerization reaction zone.

The cresol isomerization reaction conditions typically include a temperature in the range of 50 °C to 600 °C, or 100 °C to 500 °C. The cresol isomerization zone is typically operated at pressures ranging from about 0 MPa(g) to 15 MPa(g), or 0.01 MPa(g) to 10 MPa(g). The WHSV is generally in the range of 0.1 to 200 hr⁻¹, or 0.2 to 100 hr⁻¹.

Any suitable cresol isomerization catalyst can be used. The cresol isomerization catalyst is typically selected to have relatively high stability at a high activity level. Suitable cresol isomerization catalysts include, but are not limited to zeolites, acidic clay, silica alumina, acidic resins, mixed metal oxides, and the like, as are known in the art.

The transalkylated alkylphenol stream can optionally be sent to a phenolic separation zone before it is sent to the second transalkylation reaction zone. The unconverted alkylphenols having an alkyl chain of 2 or more carbon atoms can be separated into a recycle stream and recycled to the first transalkylation reaction zone.

The aromatic stream from the first transalkylation reaction zone can be sent to an aromatic separation zone where it is separated into one or more of a recycle stream comprising one or more of benzene and toluene, a heavy alkylbenzene stream comprising heavy alkylbenzenes, and a mixed xylene stream comprising xylenes and ethylbenzene. The recycle stream from the aromatic separation zone can be recycled to the first transalkylation reaction zone.

The mixed xylene stream can be sent to a xylene separation zone to be separated into a second xylene stream comprising ethylbenzene, o-xylene and m-xylene, and a p-xylene stream comprising p-xylene. The p-xylene stream can be recovered. The second xylene stream can be isomerized in a xylene isomerization reaction zone to form an isomerized xylene stream. The isomerized xylene stream can be recycled to the aromatic separation zone.

The xylene isomerization reaction conditions include a temperature is typically in the range of 50°C to 600 °C, or 100 °C to 500 °C. The xylene isomerization zone is typically operated at pressures ranging from about 0 MPa(g) to 7.6 MPa(g), or 0.01 MPa(g) to 5 MPa(g). The WHSV is generally in the range of 0.1 to 200hr⁻¹, or 0.2 to 100 hr⁻¹.

Any suitable xylene isomerization catalyst can be used. Suitable xylene isomerization catalysts include, but are not limited to homogeneous catalysts, such as BF3-HF, and heterogeneous catalysts, such as amorphous silica alumina, zeolites or metal promoted zeolites. The catalyst is typically selected to have relatively high stability at a high activity level.

The heavy alkylbenzene stream can be sent to a heavy alkylbenzene dealkylation reaction zone and/or a heavy alkylbenzene transalkylation reaction zone to make benzene, toluene, and xylenes as the main products. The treated heavy alkylbenzene stream can be sent to the aromatic separation zone.

The heavy alkylbenzene dealkylation reaction conditions include a temperature is typically in the range of 0 °C to 600 °C, or 100 °C to 500 °C. The heavy alkylbenzene dealkylation zone is typically operated at pressures ranging from about 0 MPa(g) to 7.6 MPa(g), or 0.01 MPa(g) to 5 MPa(g). The WHSV is generally in the range of 0.01 to 200 hr⁻¹, or 0.1 to 100 hr⁻¹. Any suitable heavy alkylbenzene dealkylation catalyst can be used. The catalyst is typically selected to have relatively high stability at a high activity level. Suitable heavy alkylbenzene dealkylation catalysts include, but are not limited to zeolites, acidic clay, silica alumina, acidic resins, mixed metal oxides, and the like, as are known in the art.

The heavy alkylbenzene transalkylation reaction conditions include a temperature is typically in the range of 0 °C to 600 °C, or 100 °C to 500 °C. The heavy alkylbenzene transalkylation is typically operated at pressures ranging from about _{_} 0 MPa(g) to 15 MPa(g), or 1 MPa(g) to 11 MPa(g). The WHSV is generally in the range of 0.1 to 300 hr⁻¹, or 0.2 to 200 hr⁻¹. Any suitable heavy alkylbenzene transalkylation catalyst can be used. The catalyst is typically selected to have relatively high stability at a high activity level. Suitable heavy alkylbenzene transalkylation catalysts include, but are not limited to zeolites, acidic clay, silica alumina, acidic resins, mixed metal oxides, and the like as are known in the art.

One aspect of the invention is a process for producing cresol. In one embodiment, the process comprises: providing an alkylphenol stream comprising alkylphenol; transalkylating the alkylphenol stream and a reactant stream comprising one or more of benzene and toluene in a first transalkylation reaction zone under first transalkylation reaction conditions in the presence of a first transalkylation catalyst to produce a first transalkylation effluent stream comprising at least one of phenol, cresol, xylenol, trimethylphenol, benzene, toluene, xylenes, ethylbenzene, heavy alkylbenzenes, and alkylphenol having an alkyl chain of 2 or more carbon atoms; separating the first transalkylation effluent stream in a first transalkylation separation zone into a transalkylated alkylphenol stream comprising at least one of the phenol, the cresol, the xylenol, the trimethylphenol, and the alkylphenol having the alkyl chain of 2 or more carbon atoms, and an aromatic stream comprising benzene, toluene, xylenes, ethylbenzene, and heavy alkylbenzenes; transalkylating at least a portion of the transalkylated alkylphenol stream with phenol in a second transalkylation reaction zone under second transalkylation reaction conditions in the presence of a second transalkylation catalyst to produce a second transalkylation effluent stream comprising cresol, and at least one of the unconverted phenol, xylenol, and trimethylphenol; and recovering a cresol stream comprising the cresol.

In some embodiments, the process of the invention further comprises separating the transalkylated alkylphenol stream in a phenolic separation zone into a first phenolic stream comprising the phenol, the cresol, the xylenol, and the trimethylphenol, and a second stream comprising the alkylphenols having the alkyl chain of 2 or more carbon atoms; and recycling the second stream to the first transalkylation reaction zone; wherein the at least a portion of the transalkylated alkylphenol stream comprises the first phenolic stream.

In some embodiments, recovering the cresol stream comprises separating the second transalkylation effluent stream in a second transalkylation separation zone into the cresol stream and a recycle phenol stream comprising the phenol, the xylenol, and the trimethylphenol; and recycling the recycle phenol stream to the phenolic separation zone located between the first and second transalkylation reaction zone.

In some embodiments, the process further comprises separating the aromatic stream in an aromatic separation zone into at least a recycle stream comprising one or more of benzene and toluene, a heavy alkylbenzene stream comprising the heavy alkylbenzene, and a mixed xylene stream comprising the xylene and ethylbenzene; separating the mixed xylene stream in a xylene separation zone into a second xylene stream comprising ethylbenzene, o-xylene and m-xylene, and a p-xylene stream comprising p-xylene; isomerizing the second xylene stream in a xylene isomerization reaction zone under xylene isomerization reaction conditions in the presence of a xylene isomerization catalyst to form a xylene isomerization effluent stream comprising mixed xylenes; recycling the xylene isomerization effluent stream to the aromatic separation zone; and recovering the p-xylene stream.

In some embodiments, the process further comprises at least one of: dealkylating the heavy alkylbenzene stream in a dealkylation reaction zone under dealkylation conditions in the presence of a dealkylation catalyst; and transalkylating the heavy alkylbenzene stream in a transalkylation reaction zone under transalkylation conditions in the presence of a transalkylation catalyst to form a treated heavy alkylbenzene effluent stream.

In some embodiments, the process further comprises recycling the treated heavy alkylbenzene effluent stream to the aromatic separation zone; and recycling the recycle stream from the aromatic separation zone to the first transalkylation reaction zone.

In some embodiments, the process further comprises isomerizing the cresol stream in a cresol isomerization reaction zone under cresol isomerization reaction conditions in the presence of a cresol isomerization catalyst to form a cresol isomerization effluent stream comprising mixed cresol; and recovering a m-cresol stream from the mixed cresol isomerization effluent stream.

In some embodiments, the process further comprises recycling the mixed cresol stream to the cresol isomerization reaction zone.

In some embodiments, the process further comprises separating a feed stream comprising alkylphenol into at least the alkylphenol stream comprising alkylphenols and a cresol stream comprising cresols; and introducing the cresol stream into the cresol isomerization zone.

In some embodiments, the process further comprises: treating the alkylphenol stream for removal contaminants such as nitrogen, sulfur, and other elements before introducing the alkylphenol stream into the first transalkylation zone.

In some embodiments, providing the alkylphenol stream comprises: providing a coal-derived feed stream to a first separation zone; and separating the coal-derived feed stream in the first separation zone into the alkylphenol stream and at least one of a first phenol stream comprising phenol and a first cresol stream comprising cresol.

In some embodiments, separating the coal-derived feed stream comprises separating the coal-derived feed stream by one or more of distillation, extraction, solvent extraction, acid and base extraction, adsorption, extractive distillation, crystallization, supercritical fluid extraction, chelation, eutectic reaction, or membrane separation.

In some embodiments, the coal-derived feed stream comprises a portion of a low temperature coal tar stream, a medium temperature coal tar stream, a high temperature coal tar stream, a cresylic acid stream, or a crude phenolic mixture.

In some embodiments, the first transalkylation reaction conditions or the second transalkylation reaction conditions or both comprise one or more of: a temperature in a range of 50°C to 600°C; a pressure in a range of 0 MPa(g) to 15 MPa(g); and a WHSV in a range of 0.1 to 300 hr⁻¹.

In some embodiments, the process further comprises; introducing a phenol stream comprising phenol into the second transalkylation reaction zone.

In some embodiments, transalkylating the alkylphenol stream and the reactant stream takes place in the presence of hydrogen, nitrogen, or a combination thereof in the first transalkylation reaction zone.

In some embodiments, transalkylating the at least the portion of the transalkylated alkylphenol stream with the phenol takes place in the presence of hydrogen, nitrogen, steam or a combination thereof in the second transalkylation reaction zone.

In some embodiments, the first transalkylation catalyst comprises a heterogeneous acid catalyst, wherein the second transalkylation catalyst comprises a heterogeneous acid catalyst, or both.

In some embodiments, at least one of the first transalkylation reaction zone and the second transalkylation reaction zone comprises a fixed bed reactor, a moving bed reactor, an ebullated bed reactor, a fluidized bed reactor, a continuous catalyst regeneration (CCR) reactor, a semi-regenerative reactor, a batch reactor, a continuous stirred tank (CSTR) reactor, a slurry reactor, or combinations thereof.

In some embodiments, the process further comprises separating a feed stream comprising alkylphenol into at least the alkylphenol stream comprising alkylphenols and a phenol stream comprising phenols; and introducing the phenol stream into the second transalkylation zone.

The Figure is an illustration one embodiment a process 100 according to the present invention.

The coal-derived feed stream 105 is sent to a first separation zone 110, such as a distillation column. The coal-derived feed stream 105 may be filtered and have water removed before being sent to the first separation zone 110 (not shown). The coal-derived feed stream is separated into stream 115 of hydrocarbons having a boiling point less than about 300°C and stream 120 of hydrocarbons having a boiling point greater than about 300°C.

The phenolic compounds are found in stream 115. Stream 115 is sent to a second separation zone 125, such as an extraction zone, where it is separated into a fraction 130 containing the alkylphenol compounds and stream 135 containing the non-phenolic and heavy phenolic compounds.

The fraction 130 is sent to treating zone 140 to remove the contaminants forming an alkylphenol stream 145 comprising alkylphenol. Optionally, phenol and cresols can be separated at this point in a third separation zone 146 and be sent to the second transalkylation reaction zone (stream 147 comprising phenol) and cresol separation zone (stream 148 comprising cresol), respectively.

The alkylphenol stream 149 is sent to the first transalkylation reaction zone 150 along with a reactant stream 155 comprising benzene and/or toluene. The first transalkylation reaction zone 150 contains a first transalkylation catalyst. The first transalkylation effluent stream 160 comprising phenol, cresol, xylenol, trimethylphenol, benzene, toluene, xylene, ethylbenzene, heavy alkylbenzene, and unconverted alkylphenol having an alkyl chain of 2 or more carbon atoms.

The first transalkylation effluent stream 160 is sent to a first transalkylation separation zone 165 where it is separated into a transalkylated alkylphenol stream 170 and an aromatic stream 175. The transalkylated alkylphenol stream 170 comprises phenol, cresol, xylenol, trimethylphenol, and the unconverted alkylphenol having an alkyl chain having 2 or more carbon atoms. The aromatic stream 175 comprises benzene, toluene, xylene, ethylbenzene, and heavy alkylbenzene.

The transalkylated alkylphenol stream 170 is sent to a phenolic separation zone 180 where it is separated into a first phenolic stream 185 and a second stream 190. The first phenolic stream 185 comprises phenol, cresol, xylenol, and trimethylphenol.

The second stream 190 comprises unconverted alkylphenol having an alkyl chain having 2 or more carbon atoms, such as ethylphenol, propylphenol, and butylphenol. The second stream 190 is recycled to the first transalkylation reaction zone 150.

The first phenolic stream 185 is sent to a second transalkylation reaction zone 196 along with an optional phenol stream 195, and optionally phenol from stream 147. The second transalkylation reaction zone 196 comprises a second transalkylation catalyst. The second transalkylation effluent stream 200 comprises cresol, and unconverted phenol, xylenol, and trimethylphenol.

The second transalkylation effluent stream 200 is sent to a second transalkylation separation zone 205 where it is separated into a cresol stream 210 comprising cresol and a recycle phenol stream 215 comprising the unconverted phenol, xylenol, trimethylphenol. The recycle phenol stream 215 is recycled to the phenolic separation zone 180.

Cresol stream 210 can be recovered. Optionally, cresol stream 210 can be sent to a cresol isomerization reaction zone 220 to obtain a cresol product stream 225. The cresol product stream 225 and optionally cresol stream 148 can be separated in a cresol separation zone 230 to obtain a stream 235 comprising a specific cresol, such as m-cresol, and a mixed cresol stream 240 comprising the remaining cresols. Optionally the mixed cresol stream 240 can be recycled back to the cresol isomerization reaction zone 220.

The aromatic stream 175 from the first transalkylation separation zone 165, which comprises benzene, toluene, xylene, ethylbenzene, and heavy alkylbenzene, is sent to an aromatic separation zone 250. The aromatic stream 175 is separated into a recycle stream 255, a heavy alkylbenzene stream 260, and a mixed xylene stream 265.

The recycle stream 255, which comprises benzene and/or toluene, can be recycled to the first transalkylation reaction zone 150.

The heavy alkylbenzene stream 260, which comprises heavy alkylbenzene, can be sent to a dealkylation and/or transalkylation reaction zone 270. The treated heavy alkylbenzene stream 275 is sent to the aromatic separation zone 250.

The mixed xylene stream 265, which comprises a mixture of xylenes, may be sent to a xylene isomerization zone 280. The xylene isomerization effluent 285 is sent to a xylene separation zone 290 where it is separated into a p-xylene stream 295 and a second mixed xylene stream 300. The second mixed xylene stream 300, which comprises o-xylene, m-xylene, and ethylbenzene, is sent to the aromatic separation zone 250. The p-xylene stream 295 can be recovered.

As used herein, the term "zone" can refer to an area including one or more equipment items and/or one or more sub-zones. Equipment items can include one or more reactors or reactor vessels, heaters, exchangers, pipes, pumps, compressors, and controllers, columns, and the like. Additionally, an equipment item, such as a reactor, dryer, or vessel, can further include one or more zones or sub-zones.

As depicted, process flow lines in the figures can be referred to, interchangeably, as, e.g., lines, pipes, branches, distributors, streams, effluents, feeds, products, portions, catalysts, withdrawals, recycles, suctions, discharges, and caustics.

While at least one exemplary embodiment has been presented in the foregoing detailed description of the invention, it should be appreciated that a vast number of variations exist. It should also be appreciated that the exemplary embodiment or exemplary embodiments are only examples, and are not intended to limit the scope, applicability, or configuration of the invention in any way. Rather, the foregoing detailed description will provide those skilled in the art with a convenient road map for implementing an exemplary embodiment of the invention. It being understood that various changes may be made in the function and arrangement of elements described in an exemplary embodiment without departing from the scope of the invention as set forth in the appended claims.

## Claims

1. A process for producing cresol comprising:
providing an alkylphenol stream comprising alkylphenol;
transalkylating the alkylphenol stream and a reactant stream comprising one or more of benzene and toluene in a first transalkylation reaction zone under first transalkylation reaction conditions in the presence of a first transalkylation catalyst to produce a first transalkylation effluent stream comprising at least one of phenol, cresol, xylenol, trimethylphenol, benzene, toluene, xylenes, ethylbenzene, heavy alkylbenzenes, and alkylphenol having an alkyl chain of 2 or more carbon atoms;
separating the first transalkylation effluent stream in a first transalkylation separation zone into a transalkylated alkylphenol stream comprising at least one of the phenol, the cresol, the xylenol, the trimethylphenol, and the alkylphenol having the alkyl chain of 2 or more carbon atoms, and an aromatic stream comprising benzene, toluene, xylenes, ethylbenzene, and heavy alkylbenzenes;
transalkylating at least a portion of the transalkylated alkylphenol stream with phenol in a second transalkylation reaction zone under second transalkylation reaction conditions in the presence of a second transalkylation catalyst to produce a second transalkylation effluent stream comprising cresol, and at least one of the unconverted phenol, xylenol, and trimethylphenol; and
recovering a cresol stream comprising the cresol.

2. The process of claim 1 further comprising:
separating the transalkylated alkylphenol stream in a phenolic separation zone into a first phenolic stream comprising the phenol, the cresol, the xylenol, and the trimethylphenol, and a second stream comprising the alkylphenols having the alkyl chain of 2 or more carbon atoms; and
recycling the second stream to the first transalkylation reaction zone;
wherein the at least a portion of the transalkylated alkylphenol stream comprises the first phenolic stream.

3. The process of claim 2 wherein recovering the cresol stream comprises:
separating the second transalkylation effluent stream in a second transalkylation separation zone into the cresol stream and a recycle phenol stream comprising the phenol, the xylenol, and the trimethylphenol; and
recycling the recycle phenol stream to the phenolic separation zone located between the first and second transalkylation reaction zone.

4. The process of any one of claims 1-3 further comprising:
separating the aromatic stream in an aromatic separation zone into at least a recycle stream comprising one or more of benzene and toluene, a heavy alkylbenzene stream comprising the heavy alkylbenzene, and a mixed xylene stream comprising the xylene and ethylbenzene;
separating the mixed xylene stream in a xylene separation zone into a second xylene stream comprising ethylbenzene, o-xylene and m-xylene, and a p-xylene stream comprising p-xylene;
isomerizing the second xylene stream in a xylene isomerization reaction zone under xylene isomerization reaction conditions in the presence of a xylene isomerization catalyst to form a xylene isomerization effluent stream comprising mixed xylenes;
recycling the xylene isomerization effluent stream to the aromatic separation zone; and
recovering the p-xylene stream.

5. The process of claim 4 further comprising:
at least one of: dealkylating the heavy alkylbenzene stream in a dealkylation reaction zone under dealkylation conditions in the presence of a dealkylation catalyst; and transalkylating the heavy alkylbenzene stream in a transalkylation reaction zone under transalkylation conditions in the presence of a transalkylation catalyst to form a treated heavy alkylbenzene effluent stream; optionally, the process further comprising:
recycling the treated heavy alkylbenzene effluent stream to the aromatic separation zone; and
recycling the recycle stream from the aromatic separation zone to the first transalkylation reaction zone.

6. The process of any one of claims 1-5 further comprising:
isomerizing the cresol stream in a cresol isomerization reaction zone under cresol isomerization reaction conditions in the presence of a cresol isomerization catalyst to form a cresol isomerization effluent stream comprising mixed cresol; and
recovering a m-cresol stream from the mixed cresol isomerization effluent stream; optionally, further comprising:
recycling the mixed cresol stream to the cresol isomerization reaction zone, or
separating a feed stream comprising alkylphenol into at least the alkylphenol stream comprising alkylphenols and a cresol stream comprising cresols;
and
introducing the cresol stream into the cresol isomerization zone.

7. The process of any one of claims 1-6 further comprising:
treating the alkylphenol stream for removal of contaminants before introducing the alkylphenol stream into the first transalkylation zone.

8. The process of any one of claims 1-7 wherein providing the alkylphenol stream comprises:
providing a coal-derived feed stream to a first separation zone; and
separating the coal-derived feed stream in the first separation zone into the alkylphenol stream and at least one of a first phenol stream comprising phenol and a first cresol stream comprising cresol; optionally, wherein separating the coal-derived feed stream comprises separating the coal-derived feed stream by one or more of distillation, extraction, solvent extraction, acid and base extraction, adsorption, extractive distillation, crystallization, supercritical fluid extraction, chelation, eutectic reaction, or membrane separation; and/or wherein the coal-derived feed stream comprises a portion of a low temperature coal tar stream, a medium temperature coal tar stream, a high temperature coal tar stream, a cresylic acid stream, or a crude phenolic mixture.

9. The process of any one of claims 1-8 wherein the first transalkylation reaction conditions or the second transalkylation reaction conditions or both comprise one or more of: a temperature in a range of 50°C to 600°C; a pressure in a range of 0 MPa(g) to 15 MPa(g); and a WHSV in a range of 0.1 to 300 hr⁻¹.

10. The process of any one of claims 1-9 further comprising;
introducing a phenol stream comprising phenol into the second transalkylation reaction zone.

11. The process of any one of claims 1-10 wherein transalkylating the alkylphenol stream and the reactant stream takes place in the presence of hydrogen, nitrogen, or a combination thereof in the first transalkylation reaction zone.

12. The process of any one of claims 1-11 wherein transalkylating the at least the portion of the transalkylated alkylphenol stream with the phenol takes place in the presence of hydrogen, nitrogen, steam or a combination thereof in the second transalkylation reaction zone.

13. The process of any one of claims 1-12 wherein the first transalkylation catalyst comprises a heterogeneous acid catalyst, wherein the second transalkylation catalyst comprises a heterogeneous acid catalyst, or both.

14. The process of any one of claims 1-13 wherein at least one of the first transalkylation reaction zone and the second transalkylation reaction zone comprises a fixed bed reactor, a moving bed reactor, an ebulliated bed reactor, a fluidized bed reactor, a continuous catalyst regeneration (CCR) reactor, a semi-regenerative reactor, a batch reactor, a continuous stirred tank (CSTR) reactor, a slurry reactor, or combinations thereof.

15. The process of any one of claims 1-14 further comprising:
separating a feed stream comprising alkylphenol into at least the alkylphenol stream comprising alkylphenols and a phenol stream comprising phenols; and
introducing the phenol stream into the second transalkylation zone.

## Patentansprüche

1. Verfahren zur Herstellung von Kresol, umfassend:
Bereitstellen eines Alkylphenolstroms umfassend Alkylphenol;
Transalkylieren des Alkylphenolstroms und eines Reaktantenstroms, umfassend eines oder mehrere von Benzol und/oder Toluol, in einer ersten Transalkylierungsreaktionszone unter ersten Transalkylierungsreaktionsbedingungen in Gegenwart eines ersten Transalkylierungskatalysators, um einen ersten Transalkylierungsabflussstrom zu erzeugen, umfassend mindestens eines von Phenol, Kresol, Xylenol, Trimethylphenol, Benzol, Toluol, Xylolen, Ethylbenzol, schwere Alkylbenzole und Alkylphenol mit einer Alkylkette mit 2 oder mehr Kohlenstoffatomen;
Trennen des ersten Transalkylierungsabflussstroms in einer ersten Transalkylierungstrennzone in einen transalkylierten Alkylphenolstrom, umfassend mindestens eines von Phenol, Kresol, Xylenol, Trimethylphenol und Alkylphenol mit einer Alkylkette mit 2 oder mehr Kohlenstoffatomen, und einen aromatischen Strom, umfassend Benzol, Toluol, Xylole, Ethylbenzol und schwere Alkylbenzole;
Transalkylieren von mindestens einem Teil des transalkylierten Alkylphenolstroms mit Phenol in einer zweiten Transalkylierungsreaktionszone unter zweiten Transalkylierungsreaktionsbedingungen in Gegenwart eines zweiten Transalkylierungskatalysators, um einen zweiten Transalkylierungsabflussstrom zu erzeugen, umfassend Kresol und mindestens eines von nicht umgewandeltem Phenol, Xylenol und Trimethylphenol; und
Gewinnen eines Kresolstroms umfassend Kresol.

2. Verfahren nach Anspruch 1, ferner umfassend:
Trennen des transalkylierten Alkylphenolstroms in einer Phenoltrennzone in einen ersten Phenolstrom, das Phenol, umfassend das Kresol, das Xylenol und das Trimethylphenol, und einen zweiten Strom, umfassend die Alkylphenole mit der Alkylkette aus 2 oder mehr Kohlenstoffatomen; und
Rückführen des zweiten Stroms zu der ersten Transalkylierungsreaktionszone;
Wobei der mindestens eine Teil des transalkylierten Alkylphenolstroms den ersten Phenolstrom umfasst.

3. Verfahren nach Anspruch 2, wobei die Rückgewinnung des Kresolstroms umfasst:
Auftrennen des zweiten Transalkylierungsabflussstroms in einer zweiten Transalkylierungstrennzone in den Kresolstrom und einen Rückführphenolstrom, umfassend das Phenol, das Xylenol und das Trimethylphenol; und
Rückführen des Rückführphenolstroms in die Phenoltrennzone, die zwischen der ersten und zweiten Transalkylierungsreaktionszone liegt.

4. Verfahren nach einem der Ansprüche 1 bis 3, ferner umfassend: Trennen des aromatischen Stroms in einer Aromatentrennzone in mindestens eines von
einem Rückführstrom, umfassend eines oder mehrere von Benzol und Toluol, von einem Strom aus schwerem Alkylbenzol, umfassend das schwere Alkylbenzol, und von einem gemischten Xylolstrom, umfassend Xylol und Ethylbenzol;
Trennen des gemischten Xylolstroms in einer Xyloltrennzone in einen zweiten Xylolstrom, umfassend Ethylbenzol, o-Xylol und m-Xylol, und einen p-Xylolstrom, umfassend p-Xylol;
Isomerisieren des zweiten Xylolstroms in einer
Xylolisomerisierungsreaktionszone unter Xylolisomerisierungsreaktionsbedingungen in Gegenwart eines Xylolisomerisierungskatalysators, um einen Xylolisomerisierungsabflussstrom zu bilden, umfassend gemischte Xylole;
Rückführen des Xylolisomerisierungsabflussstroms zu der Aromatentrennzone; und
Rückgewinnung des p-Xylolstroms.

5. Verfahren nach Anspruch 4, ferner umfassend:
mindestens eines von: Dealkylieren des schweren Alkylbenzolstroms in einer Dealkylierungsreaktionszone unter Dealkylierungsbedingungen in Gegenwart eines Dealkylierungskatalysators; und Transalkylieren des schweren Alkylbenzolstroms in einer Transalkylierungsreaktionszone unter Transalkylierungsbedingungen in Gegenwart eines Transalkylierungskatalysators, um einen behandelten schweren Alkylbenzol-Abflussstrom zu bilden; das Verfahren optional ferner umfassend:
Rückführen des behandelten schweren Alkylbenzolabflussstroms in die Aromatentrennzone; und
Rückführen des Rückführstroms von der Aromatentrennzone zu der Transalkylierungsreaktionszone.

6. Verfahren nach einem der Ansprüche 1 bis 5, ferner umfassend:
Isomerisieren des Kresolstroms in einer Kresolisomerisierungsreaktionszone unter Kresolisomerisierungsreaktionsbedingungen in Gegenwart eines Kresolisomerisierungskatalysators, um einen Kresolisomerisierungsabflussstrom zu bilden, umfassend gemischtes Kresol; und
Rückgewinnung eines m-Kresolstroms aus dem gemischten Kresolisomerisierungsabflussstrom; optional ferner umfassend:
Rückführen des gemischten Kresolstroms in die Kresolisomerisierungsreaktionszone oder
Auftrennen eines Alkylphenol umfassenden Zufuhrstroms in mindestens den Alkylphenole umfassenden Alkylphenolstrom und einen Kresole umfassenden Kresolstrom;
und
Einführen des Kresolstroms in die Kresolisomerisierungszone.

7. Verfahren nach einem der Ansprüche 1 bis 6, ferner umfassend:
Behandeln des Alkylphenolstroms zur Entfernung von Verunreinigungen vor dem Einführen des Alkylphenolstroms in die erste Transalkylierungszone.

8. Verfahren nach einem der Ansprüche 1 bis 7, wobei das Bereitstellen des Alkylphenolstroms umfasst:
Bereitstellen eines aus Kohle gewonnenen Zufuhrstroms für eine erste Trennzone; und
Trennen des aus Kohle gewonnenen Zufuhrstroms in der ersten Trennzone in den Alkylphenolstrom und mindestens einen von einem ersten Phenolstrom, umfassend Phenol, und einen ersten Kresolstrom, umfassend Kresol; optional, wobei das Trennen des aus Kohle gewonnenen Zufuhrstroms das Trennen des aus Kohle gewonnenen Zufuhrstroms durch eines oder mehrere von Destillation, Extraktion, Lösungsmittelextraktion, Säure- und Basenextraktion, Adsorption, Extraktivdestillation, Kristallisation, Extraktion mit überkritischen Fluiden, Chelatisierung, eutektische Reaktion oder Membrantrennung umfasst; und/oder wobei der aus Kohle gewonnene Zufuhrstrom einen Teil eines Niedertemperatur-Kohlenteerstroms, eines Mitteltemperatur-Kohlenteerstroms, eines Hochtemperatur-Kohlenteerstroms, eines Kresylsäurestroms oder einer rohen Phenolmischung umfasst.

9. Verfahren nach einem der Ansprüche 1 bis 8, wobei die ersten Transalkylierungsreaktionsbedingungen oder die zweiten Transalkylierungsreaktionsbedingungen oder beide eines oder mehrere umfassen von: eine Temperatur in einem Bereich von 50 °C bis 600 °C; einen Druck in einem Bereich von 0 MPa(g) bis 15 MPa(g); und eine WHSV in einem Bereich von 0,1 bis 300 h⁻¹.

10. Verfahren nach einem der Ansprüche 1 bis 9, ferner umfassend;
Einführen eines Phenolstroms, umfassend Phenol, in die zweite Transalkylierungsreaktionszone.

11. Verfahren nach einem der Ansprüche 1 bis 10, wobei die Transalkylierung des Alkylphenolstroms und des Reaktantenstroms in Gegenwart von Wasserstoff, Stickstoff oder einer Kombination davon in der ersten Transalkylierungsreaktionszone stattfindet.

12. Verfahren nach einem der Ansprüche 1 bis 11, wobei die Transalkylierung mindestens des Teils des transalkylierten Alkylphenolstroms mit dem Phenol in Gegenwart von Wasserstoff, Stickstoff, Dampf oder einer Kombination davon in der zweiten Transalkylierungsreaktionszone stattfindet.

13. Verfahren nach einem der Ansprüche 1 bis 12, wobei der erste Transalkylierungskatalysator einen heterogenen Säurekatalysator umfasst, wobei der zweite Transalkylierungskatalysator einen heterogenen Säurekatalysator umfasst oder beides.

14. Verfahren nach einem der Ansprüche 1 bis 13, wobei mindestens eine der ersten Transalkylierungsreaktionszone und der zweiten Transalkylierungsreaktionszone einen Festbettreaktor, einen Wanderbettreaktor, einen Siedebettreaktor, einen Wirbelschichtreaktor, einen Reaktor zur kontinuierlichen Katalysatorregeneration (CCR, continuous catalyst regeneration reactor), einen halbregenerativen Reaktor, einen Batch-Reaktor, einen kontinuierlich gerührten Tankreaktor (CSTR, continuous stirred tank reactor), einen Suspensionsreaktor oder Kombinationen davon umfasst.

15. Verfahren nach einem der Ansprüche 1 bis 14, ferner umfassend:
Auftrennen eines Alkylphenol umfassenden Zufuhrstroms in mindestens den Alkylphenole umfassenden Alkylphenolstrom und einen Phenole umfassenden Phenolstrom; und
Einführen des Phenolstroms in die zweite Transalkylierungszone.

## Revendications

1. Procédé permettant de produire du crésol comprenant :
la fourniture d'un flux d'alkylphénol comprenant de l'alkylphénol ;
la transalkylation du flux d'alkylphénol et d'un flux de réactif comprenant un ou plusieurs parmi le benzène et le toluène dans une première zone de réaction de transalkylation dans des conditions de première réaction de transalkylation en présence d'un premier catalyseur de transalkylation pour produire un premier flux d'effluent de transalkylation comprenant au moins l'un parmi du phénol, crésol, xylénol, triméthylphénol, benzène, toluène, des xylènes, de l'éthylbenzène, des alkylbenzènes lourds et de l'alkylphénol ayant une chaîne d'alkyle de 2 atomes de carbone ou plus ;
la séparation du premier flux d'effluent de transalkylation dans une première zone de séparation de transalkylation en un flux d'alkylphénol transalkylé comprenant au moins l'un parmi le phénol, le crésol, le xylénol, le triméthylphénol et l'alkylphénol ayant une chaîne alkyle de 2 atomes de carbone ou plus, et un flux aromatique comprenant du benzène, du toluène, des xylènes, de l'éthylbenzène et des alcoylbenzènes lourds ;
la transalkylation d'au moins une partie du flux d'alkylphénol transalkylé avec du phénol dans une seconde zone de réaction de transalkylation dans des conditions de seconde réaction de transalkylation en présence d'un second catalyseur de transalkylation pour produire un second flux d'effluent de transalkylation comprenant du crésol et au moins l'un parmi le phénol non converti, le xylénol et le triméthylphénol ; et
la récupération d'un flux de crésol comprenant le crésol.

2. Procédé selon la revendication 1, comprenant en outre :
la séparation du flux d'alkylphénol transalkylé dans une zone de séparation phénolique en un premier flux phénolique comprenant le phénol, le crésol, le xylénol et le triméthylphénol, et un second flux comprenant les alkylphénols ayant la chaîne alkyle de 2 atomes de carbone ou plus ; et
le recyclage du second flux vers la première zone de réaction de transalkylation ;
dans lequel l'au moins une partie du flux d'alkylphénol transalkylé comprend le premier flux phénolique.

3. Procédé selon la revendication 2 dans lequel la récupération du flux de crésol comprend :
la séparation du second flux d'effluent de transalkylation dans une seconde zone de séparation de transalkylation en flux de crésol et un flux de phénol de recyclage comprenant le phénol, le xylénol et le triméthylphénol ; et
le recyclage du flux de phénol recyclé dans la zone de séparation phénolique située entre les première et seconde zones de réaction de transalkylation.

4. Procédé selon l'une quelconque des revendications 1 à 3 comprenant en outre : la séparation du flux aromatique dans une zone de séparation aromatique en au moins
un flux de recyclage comprenant un ou plusieurs parmi le benzène et le toluène, un flux d'alkylbenzène lourd comprenant l'alkylbenzène lourd, et un flux de xylène mélangé comprenant le xylène et l'éthylbenzène ;
la séparation du flux de xylène mélangé dans une zone de séparation de xylène en un second flux de xylène comprenant de l'éthylbenzène, de l'o-xylène et du m-xylène, et un flux de p-xylène comprenant du p-xylène ;
l'isomérisation du second flux de xylène dans une zone de réaction d'isomérisation de xylène dans des conditions de réaction d'isomérisation de xylène en présence d'un catalyseur d'isomérisation de xylène pour former un flux d'effluent d'isomérisation de xylène comprenant des xylènes mélangés ;
le recyclage du flux d'effluent d'isomérisation de xylène vers la zone de séparation aromatique. et
la récupération du flux de p-xylène.

5. Procédé selon la revendication 4, comprenant en outre :
au moins l'un parmi : la désalkylation du flux d'alkylbenzène lourd dans une zone de réaction de désalkylation dans des conditions de désalkylation en présence d'un catalyseur de désalkylation ; et la transalkylation du flux d'alkylbenzène lourd dans une zone de réaction de transalkylation dans des conditions de transalkylation en présence d'un catalyseur de transalkylation pour former un flux d'effluent d'alkylbenzène lourd traité ; éventuellement, le procédé comprenant en outre :
le recyclage du flux d'effluent d'alkylbenzène lourd traité dans la zone de séparation aromatique ; et
le recyclage du flux recyclé de la zone de séparation aromatique vers la première zone de réaction de transalkylation

6. Procédé selon l'une quelconque des revendications 1 à 5, comprenant en outre :
l'isomérisation du flux de crésol dans une zone de réaction d'isomérisation de crésol dans des conditions de réaction d'isomérisation de crésol en présence d'un catalyseur d'isomérisation de crésol pour former un flux d'effluent d'isomérisation de crésol comprenant du crésol mélangé ; et
la récupération d'un flux de m-crésol à partir du flux d'effluent de d'isomérisation de crésol mélangé ; éventuellement, comprenant en outre :
le recyclage du flux de crésol mélangé dans la zone de réaction d'isomérisation de crésol, ou
la séparation d'un flux d'alimentation comprenant de l'alkylphénol en au moins le flux d'alkylphénol comprenant des alkylphénols et un flux de crésol comprenant des crésols ;
et
l'introduction du flux de crésol dans la zone d'isomérisation de crésol.

7. Procédé selon l'une quelconque des revendications 1 à 6, comprenant en outre :
le traitement du flux d'alkylphénol pour éliminer des contaminants avant l'introduction du flux d'alkylphénol dans la première zone de transalkylation.

8. Procédé selon l'une quelconque des revendications 1 à 7, dans lequel le flux d'alkylphénol comprend :
la fourniture d'un flux d'alimentation dérivé du charbon à une première zone de séparation ; et
la séparation du flux d'alimentation dérivé du charbon dans la première zone de séparation en flux d'alkylphénol et au moins l'un parmi un premier flux de phénol comprenant du phénol et un premier flux de crésol comprenant du crésol ; éventuellement, dans lequel la séparation du flux d'alimentation dérivé du charbon comprend la séparation du flux d'alimentation dérivé du charbon par un ou plusieurs parmi la distillation, l'extraction, l'extraction par solvant, l'extraction acide et basique, l'adsorption, la distillation extractive, la cristallisation, l'extraction par fluide supercritique, la chélation, la réaction eutectique ou la séparation par membrane ; et/ou dans lequel le flux d'alimentation dérivé du charbon comprend une partie d'un flux de goudron de houille à basse température, d'un flux de goudron de houille à température moyenne, d'un flux de goudron de houille à haute température, d'un flux d'acide crésylique ou d'un mélange phénolique brut.

9. Procédé selon l'une quelconque des revendications 1 à 8, dans lequel les conditions de première réaction de transalkylation ou les conditions de seconde réaction de transalkylation, ou les deux, comprennent une ou plusieurs parmi : une température dans une plage comprise entre 50 °C et 600 °C ; une pression dans une plage comprise entre 0 MPa(g) et 15 MPa(g) ; et une WHSV dans une plage comprise entre 0,1 et 300 hr'¹.

10. Procédé selon l'une quelconque des revendications 1 à 9, comprenant en outre ;
l'introduction d'un flux de phénol comprenant du phénol dans la seconde zone de réaction de transalkylation.

11. Procédé selon l'une quelconque des revendications 1 à 10 dans lequel la transalkylation du flux d'alkylphénol et du flux de réactif a lieu en présence d'hydrogène, d'azote ou d'une combinaison de ceux-ci dans la première zone de réaction de transalkylation.

12. Procédé selon l'une quelconque des revendications 1 à 11 dans lequel la transalkylation de l'au moins une partie du flux d'alkylphénol transalkylé avec le phénol a lieu en présence d'hydrogène, d'azote, de vapeur ou d'une combinaison de ceux-ci dans la seconde zone de réaction de transalkylation.

13. Procédé selon l'une quelconque des revendications 1 à 12, dans lequel le premier catalyseur de transalkylation comprend un catalyseur acide hétérogène, dans lequel le second catalyseur de transalkylation comprend un catalyseur acide hétérogène, ou les deux.

14. Procédé selon l'une quelconque des revendications 1 à 13 dans lequel au moins l'une parmi la première zone de réaction de transalkylation et la seconde zone de réaction de transalkylation comprend un réacteur à lit fixe, un réacteur à lit mobile, un réacteur à lit bouillonnant, un réacteur à lit fluidisé, un réacteur de régénération catalytique continue (CCR), un réacteur semi-régénératif, un réacteur discontinu, un réacteur à agitation continue (CSTR), un réacteur à combustible en suspension, ou des combinaisons de ceux-ci.

15. Procédé selon l'une quelconque des revendications 1 à 14, comprenant en outre :
la séparation d'un flux d'alimentation comprenant de l'alkylphénol en au moins un flux d'alkylphénol comprenant des alkylphénols et un flux de phénol comprenant des phénols ; et
l'introduction du flux de phénol dans la seconde zone de transalkylation.
